(19) Europäisches Patentamt
European Patent Office
Office européen des brevets

(11) Numéro de publication: **0 000 681**
**B1**

(12) **FASCICULE DE BREVET EUROPEEN**

(45) Date de publication du fascicule de brevet: **26.08.81**

(21) Numéro de dépôt: **78400062.2**

(22) Date de dépôt: **21.07.78**

(51) Int. Cl.³: **C 07 D 239/48,**
A 01 N 43/54 //C07D239/46

(54) **Amino-2 (ou -4) alkylthio-5 pyrimidines, leurs procédés de préparation, leur application comme herbicides et compositions les contenant.**

(30) Priorité: **28.07.77 FR 7723222**
**05.02.78 FR 7804207**

(43) Date de publication de la demande:
**07.02.79 Bulletin 79/3**

(45) Mention de la délivrance du brevet:
**26.08.81 Bulletin 81/34**

(84) Etats Contractants Désignés:
**BE CH DE FR GB LU NL**

(56) Documents cités:
**BE - A - 841 390**
**FR - A - 2 119 234**
**FR - A - 2 173 746**

(73) Titulaire: **P C U K PRODUITS CHIMIQUES UGINE KUHLMANN**
**Service Propriété Industrielle Tour Manhattan**
**F-92087 PARIS LA DEFENSE 2 Cédex 21 (FR)**

(72) Inventeur: **Balde, Daniel**
**13 rue Charles Friedel**
**F-75020 Paris (FR)**
Inventeur: **Boutemy, Gérard Emile Marcel**
**Le Clos d'Artois Oncy-sur-Ecole**
**F-91490 Milly la Foret (FR)**

(74) Mandataire: **Houssin, Jean Produits Chimiques Ugine Kuhlmann et al,**
**Service Propriété Industrielle Tour Manhattan**
**Cedex 21**
**F-92087 Paris la Defense (FR)**

**EP 0 000 681 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Amino-2 (ou -4) alkylthio-5 pyrimidines, leurs procédés de préparation, leur application comme herbicides et compositions les contenant

La présente invention a pour objet de nouvelles alkylthio-5 pyrimidines portant un groupe amino ou acylamino, leurs procédés de préparation et leur application en tant qu'herbicides.

Il est déjà connu des dérivés de la pyrimidine herbicides (voir par exemple les brevets français 2 031 422, 2 317 291, 2 119 234 et 2 137 933), mais ces dérivés ne portent jamais simultanément un groupe alkylthio en position 5 et un groupe amino ou acylamino.

Les nouvelles alkylthio-5 pyrimidines selon l'invention peuvent être représentées par la formule générale:

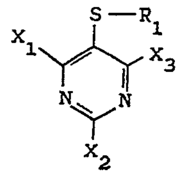

(I)

dans laquelle $R_1$ est un groupe alkyle ayant 1 à 5 atomes de carbone, $X_1$ est un atome de chlore, $X_2$ est un groupe

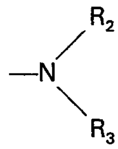

dans lequel $R_2$ est un atome d'hydrogène ou un groupe alkyle ayant 1 à 5 atomes de carbone, $R_3$ est un atome d'hydrogène ou un groupe alkyle ayant 1 à 5 atomes de carbone et peut être en outre, lorsque $R_2$ est un atome d'hydrogène, un groupe

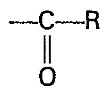

dans lequel R est un atome d'hydrogène ou un groupe alkyle de 1 à 5 atomes de carbone, $X_3$ est un groupe

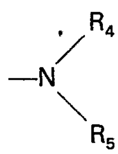

dans lequel $R_4$ est un atome d'hydrogène et $R_5$ est un atome d'hydrogène ou un groupe

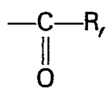

R étant tel que défini ci-dessus.

Les composés de formule (I) dans lesquels $R_3$ et $R_5$ ne sont pas

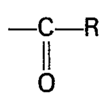

peuvent être préparés par condensation d'une trichloro-2,4,6 alkylthio-5 pyrimidine de formule (II), dans laquelle X est un atome de chlore et $R_1$ est un groupe alkyle ayant 1 à 5 atomes de carbone, avec un composé de formule (III), dans laquelle $R_2$ et $R_3$ ont les mêmes significations que dans la formule (I) excepté la signification

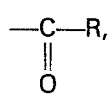

et condensation de la dichloro-4,6 alkylthio-5 pyrimidine de formule (IV) ainsi obtenue avec un composé de formule (V), dans laquelle $R_4$ et $R_5$ ont les mêmes significations que dans la formule (I) excepté la signification

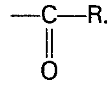

L'ensemble du processus réactionnel peut être schématisé comme suit:

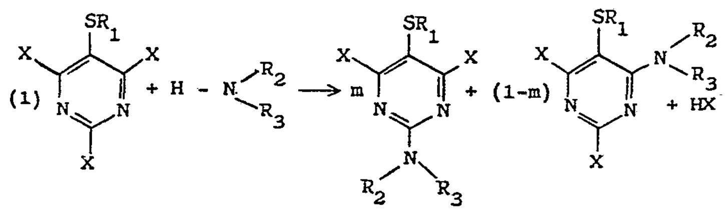

(II) (III) (IV) (IV) bis

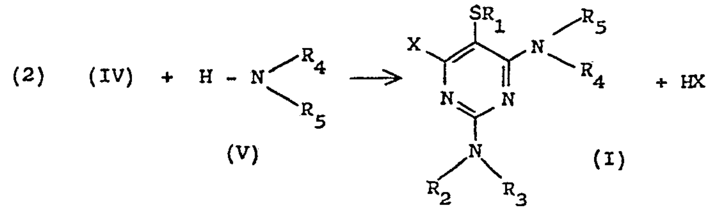

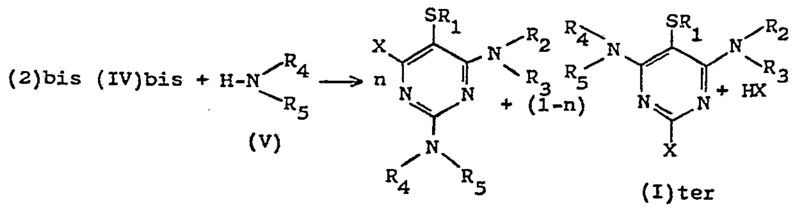

(I) bis

Dans les réactions ci-dessus, m et n sont des nombres supérieurs à O et inférieurs à 1.

Les trichloro-2,4,6 alkylthio-5 pyrimidines de formule (II) sont des produits connus. Elles peuvent être préparées par exemple par le procédé de décrit dans le brevet français 1 549 494 demandé le 31 Octobre 1967.

Les réactions de condensation (1), (2) et (2)bis peuvent être effectuées soit en milieu aqueux, soit en milieu solvant organique, soit encore dans un milieu mixte eau + solvant organique. Comme solvants organiques utilisables on peut citer en particulier, sans que cela soit limitatif, le toluène, le méthanol, des cétones aliphatiques comme l'acétone, la méthyléthylcétone ou la diéthylcétone, le diméthylformamide ou un excès du composé (III), lorsque celui-ci est une amine.

Les réactions de condensation (1), (2) et (2)bis sont effectuées en présence d'un agent basique susceptible de fixer l'acide chlorhydrique HX formé dans la réaction. Comme agents basiques utilisables on peut citer, par exemple, les hydroxydes alcalins, l'ammoniaque, ou un excès des composés de formule (III) ou (V).

Les réactions (1), (2) et (2)bis sont effectuées à une température qui est fonction en particulier du solvant utilisé. De manière générale la réaction (1) est effectuée entre 0 et 150°C. Elle peut donc être réalisée à une température inférieure à la température ordinaire, par exemple entre 0 et 10°C, ou à une température supérieure à la température ordinaire, par exemple entre 100 et 150°C. Les réactions (2) et (2) bis ne peuvent être réalisées à des températures aussi basses que celles utilisables pour la réaction (1). Elles sont effectuées en général entre 100 et 150°C. Selon la température et le solvant utilisés, les réactions (1), (2) et (2) bis sont effectuées à la pression atmosphérique ou sous une pression supérieure à la pression atmosphérique.

Les dichloro alkylthio-5 pyrimidines isomères (IV) et (IV) bis obtenues dans la réaction (1) peuvent être séparées, par exemple par cristallisation fractionnée. Les isomères ainsi séparés fournissent ensuite, par les réactions (2) et (2) bis, le composé (I) pur et le mélange des deux composés (I) bis et (I) ter, isomères du composé (I).

On peut également soumettre le mélange des composés (IV) et (IV) bis obtenu dans la réaction (1) à la deuxième étape du procédé [réaction avec le composé (V)]. On obtient alors un mélange des trois composés isomères (I), (I) bis et (I) ter, mélange qui peut être employé tel quel dans les applications

herbicides. Les composés isomères (I), (I) bis et (I) ter peuvent aussi être séparés par chromatographie préparative en phase liquide.

Dans le cas où les composés (III) et (V) sont identiques et sont donc tous les deux l'ammoniac, les isomères (I) et (I) bis sont identiques et l'ensemble du schéma réactionnel précédent conduit donc à deux isomères [isomères de formules (VI) et (VII) ci-dessous]. Dans ce même cas, et à condition d'opérer à température suffisamment élevée (100 à 150°C dans la pratique), on peut obtenir en une seule étape, à partir de la trichloro-2,4,6 alkylthio-5 pyrimidine de formule (II), un mélange des isomères (VI) et (VII), suivant la réaction:

(3)

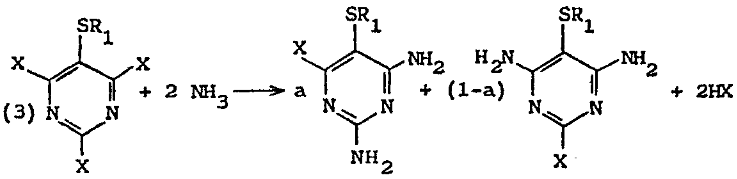

(II) (VI) (VII)

Dans ce mélange l'isomère (VI) est prépondérant (a inférieur à 1 et supérieur à 0,5).

Les composés de formule (I) dans lesquels $X_3$ est un groupe $NH_2$ et $X_2$ est un groupe

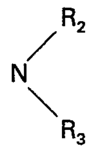

dans lequel $R_2$ et $R_3$ sont des groupes alkyle identiques R' peuvent aussi être préparés par réaction d'une trichloro-2,4,6 alkylthio-5 pyrimidine de formule (II) avec une amine tertiaire de formule (VIII) et condensation de la dichloro-4,6 alkylthio-5 pyrimidine de formule (IX) ainsi obtenue avec l'ammoniac, suivant le schéma réactionnel suivant:

(4) (II) + N (R')$_3$

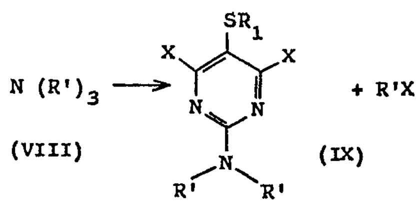

(VIII) (IX)

(5) (IX) + $NH_3$

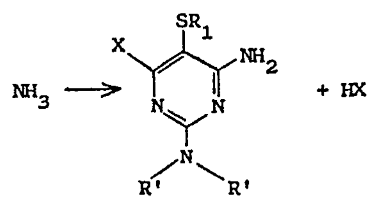

La réaction (4), qui présente l'originalité de fournir sélectivement l'isomère dichloro-4,6 alkylthio-5 pyrimidine, peut être effectuée en milieu solvant organique, à une température comprise entre 100 et 150°C. Comme solvants organiques utilisables on peut citer les mêmes solvants que pour les réactions (1), (2) et (2) bis.

La réaction (5) est réalisée dans les mêmes conditions que la réaction (2).

Les composés formés dans les réactions (1), (2), (2) bis, (3), (4) et (5) peuvent être isolés du milieu réactionnel par des méthodes chassiques telles que, par exemple, la filtration, lorsque les composés précipitent, ou la distillation sous pression réduite du solvant suivie du lavage à l'eau du résidu, et purifiés par recristallisation dans un solvant approprié.

Les composés de formule (I) dans lesquels l'un au moins des substituants $R_3$ et $R_5$ est un groupe

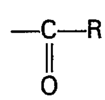

peuvent être préparés par acylation des composés de formule (I) dans lesquels $R_3$ et $R_5$ ne sont pas

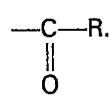

Cette acylation est effectuée à l'aide des agents d'acylation habituels tels que chlorures d'acide, anhydrides d'acide, cétène ou composés homologues. On opère en milieu solvant organique, à une température comprise entre 20 et 120°C, de préférence entre 50 et 100°C. Comme solvants organiques utilisables on peut citer en particulier les acides carboxyliques, dans le cas où on effectue l'acylation avec un anhydride d'acide, et la pyridine, dans le cas où on effectue l'acylation avec un chlorure d'acide.

Les composés de formule (I) peuvent être transformés en leurs sels avec les acides minéraux ou organiques par réaction avec l'acide correspondant au sein d'un solvant approprié.

Les composés de formule (I) et leurs sels avec les acides minéraux ou organiques ont la propriété de détruire un grand nombre de plantes indésirables appartenant aux classes des monocotylédones ou des dicotylédones et ce à des doses très faibles comprises entre 150 g/ha et 2500 g/ha. En particulier ils détruisent totalement les plantes suivantes: ray grass, panic, digitaire, sétaire, vulpin, folle avoine, gaillet, amarante, renouée, capselle, véronique, moutarde, datura, mouron, stellaire, chardon, fumeterre, chenopode, oseille, plantain, atriplex, pissenlit, coquelicot, chrysanthème, seneçon, laiteron, euphorbe. En outre, aux doses auxquelles ils sont actifs vis-à-vis des plantes indésirables, les composés de formule (I) et leurs sels n'ont en général pas d'action défavorable sur des céréales d'hiver et de printemps telles que le blé et l'orge, sur le riz et le maïs.

Les composés de formule (I) et leurs sels sont actifs vis-à-vis des plantes adventices aussi bien dans les traitements de pré-levée que dans les traitements de post-levée. Toutefois leur activité est plus marquée dan les traitements de post-levée.

Pour leur mise en oeuvre les composés herbicides selon l'invention peuvent être incorporés, conjointement avec d'autres herbicides ou séparément, dans des formulations qui contiennent, outre la matière active, les additifs inertes habituellement utilisés en agriculture pour faciliter la conservation, la mise en suspension aqueuse, l'adhérence sur le feuillage et la résistance aux agents atmosphériques et aux dégradations biologiques (d'où une persistance plus grande de l'action), tels que diluants solides (talc, silice, kieselguhr, argile, etc...) ou liquides (huiles minérales, eau, solvants organiques comme par exemple des cétones, des alcools, des hydrocarbures ou leurs dérivés chlorés), adjuvants, tensio-actifs, antioxydants et stabilisants. De telles formulations peuvent se présenter sous la forme de poudres mouillables, solutions émulsifiables dans l'eau, suspensions, granulés ou toute autre forme en usage dans le domaine des herbicides.

Dans les formulations contenant seulement des composés herbicides selon l'invention et des additifs inertes, la teneur en composés de formule (I) ou leurs sels (matière active) peut varier de 1% à 95% en poids. Dans les formulations contenant des composés herbicides selon l'invention, d'autres herbicides et des additifs inertes, la teneur en composés selon l'invention peut varier de 1% à 80% en poids, celle en herbicides autres de 80% à 1% en poids, le complément à 100% étant constitué par les additifs inertes.

Comme herbicides autres qui peuvent être associés dans les formulations aux composés selon l'invention on peut citer la (dichloro-3,4 phényl)-3 diméthyl-1,1 urée (diuron), la phényl-3 diméthyl-1,1 urée (fénuron), la (chloro-3 méthyl-4 phényl)-3 diméthyl-1,1 urée (chlortoluron), la (chloro-4 phényl)-3 diméthyl-1,1 urée (monuron), le monolinuron, la (dichloro-3,4 phényl)-3 méthoxy-1 méthyl-1 urée (linuron), l'isoproturon, le mithabenzthiazuron, la (dichloro-3,4 phényl)-3 n-butyl-1 méthyl-1 urée (néburon), la chloro-2 éthylamino-4 isopropylamino-6 triazine-1,3,5 (atrazine), la chloro-2 bis (éthylamino)-4,6 triazine-1,3,5 (simazine), l'amino-3 triazole-1,2,4, la terbutryne, la cyanazine, la diéthyl-2,6 N-chloroacétyl N-méthoxyméthyl aniline (alachlor), la N-chloroacétyl N-isopropyl aniline (propachlor), le napropamide, le diquat, le paraquat, l'acide dichloro-2,4 phénoxyacétique (2,4-D), l'acide (méthyl-2 chloro-4 phénoxy)-2 propionique (MCPP), l'acide méthoxy-2 dichloro-3,6 benzoïque (dicamba), l'acide amino-4 trichloro-3,5,6 picolinique (picloram), le dinitro-2,4 sec-butyl-6 phénol (dinoseb), le dinitro-4,6 ortho-crésol (DNOC), le N-(chloro-3 phényl) carbamate de chloro-4 butynyle-2 (barban), le propham, le terbacile, le bromo-5 sec-butyl-3 méthyl-6 uracile (bromacile), le pyrazone, le phenmedipham et le métamitron.

Les exemples suivants illustrent l'invention sans la limiter.

Exemple 1.

Amino-4 chloro-6 éthylamino-2 méthylthio-5 pyrimidine.
La préparation de ce composé est réalisée en 2 étapes.

1ère étape:

Dans un réacteur de 250 ml muni d'une agitation, on introduit 45,9 g de trichloro-2,4,6 méthylthio-5 pyrimidine, 150 g de méthyléthylcétone et 130 g d'eau. Dans ce mélange maintenu à 5°C, on ajoute en 30 minutes 9,44 g d'éthylamine en solution aqueuse à 32,5%. On maintient 1h30 à 5°C. On ajoute ensuite 8,08 g de soude en solution aqueuse à 30%. On maintient le mélange 4 heures à 20°C et une nuit au réfrigérateur à environ 0°C. Il apparait un précipité qui est isolé par filtration, lavé avec 16 ml de méthyléthylcétone et recristallisé deux fois dans 180 ml d'éthanol. On obtient ainsi 10,5 g d'éthylamino-2 dichloro-4,6 méthyl-thio-5 pyrimidine, qui fond à 147°C et qui est identifiée par son spectre dans l'infra-rouge (IR) et son spectre de résonance magnétique nucléaire (R.M.N.)

Le filtrat est évaporé. On obtient ainsi 36,5 g d'un mélange d'éthylamino-2 dichloro-4,6 méthylthio-5 pyrimidine et d'éthylamino-4 dichloro-2,6 méthylthio-5 pyrimidine, mélange qui fond à 76°C et qui est identifié par ses spectres IR et R.M.N.

2ème étape:

Dans un autoclave de 500 ml, on introduit 7,5 g d'éthylamino-2 dichloro-4,6 méthylthio-5 pyrimidine obtenue comme indiqué ci-dessus, 110 ml de méthanol et 11 g d'ammoniac. Le mélange est chauffé à 110°C pendant 2 heures puis refroidi. On concentre sous pression réduite la solution obtenue et lave à l'eau le résidu. On obtient ainsi 8,2 g d'amino-4 chloro-6 éthylamino-2 méthylthio-5 pyrimidine, qui est identifiée par ses spectres I.R. et R.M.N. et qui fond à 124°C.

Exemple 2.

Mélange des trois isomères amino-4 chloro-6 éthylamino-2 méthylthio-5 pyrimidine, amino-2 chloro-6 éthylamino-4 méthylthio-5 pyrimidine et chloro-2 amino-6 éthylamino-4 méthylthio-5 pyrimidine.

Dans un autoclave de 500 ml, on introduit 2,5 g d'éthylamino-2 dichloro-4,6 méthylthio-5 pyrimidine, 9 g du mélange d'éthylamino-2 dichloro-4,6 méthylthio-5 pyrimidine et d'éthylamino-4 dichloro-2,6 méthylthio-5 pyrimidine obtenu à la première étape de l'exemple 1, 165 g de méthanol et 16 g d'ammoniac. Le mélange est chauffé 2 heures à 120°C puis refroidi. On concentre sous pression réduite la solution obtenue et lave à l'eau le résidu. On obtient ainsi 8,2 g d'un mélange fondant à 76°C. L'analyse de ce mélange par chromatographie en phase gazeuse et spectrométrie de masse montre qu'il contient 56,6 % d'amino-2 chloro-6 éthylamino-4 méthylthio-5 pyrimidine, 37,8% d'amino-4 éthylamino-2 chloro-6 méthylthio-5 pyrimidine et 5,6% d'amino-6 éthylamino-4 chloro-2 méthylthio-5 pyrimidine.

Exemple 3.

Mélange des trois isomères amino-2 chloro-6 isopropylamino-4 méthylthio-5 pyrimidine, amino-4 chloro-6 isopropylamino-2 méthylthio-5 pyrimidine et amino-6 isopropylamino-4 chloro-2 méthylthio-5 pyrimidine.

La synthèse est réalisée en 2 étapes.

1ère étape:

On opère comme dans la première étape de l'exemple 1 en remplaçant l'éthylamine par 11,8 g d'isopropylamine. Par évaporation sous pression réduite de la solution finale, lavage à l'eau du résidu et séchage sous pression réduite, on obtient 51,4 g d'une pâte constituée par un mélange de dichloro-4,6 isopropylamino-2 méthylthio-5 pyrimidine et de dichloro-2,6 isopropylamino-4 méthylthio-5 pyrimidine.

2ème étape:

Dans un autoclave de 500 ml, on introduit 12,5 g du mélange obtenu á la premiére étape, 250 g de méthanol et 25 g d'ammoniac. Après 2 heurs de chauffage à 130°C, on concentre sous vide la solution obtenue et lave à l'eau le résidu. On obtient ainsi 12,1 g d'un produit pâteux qui, ainsi que le montrent la chromatographie en phase gazeuse et la spectrométrie de masse, est un mélange contenant 55,1% d'amino-2 chloro-6 isopropylamino-4 méthylthio-5 pyrimidine, 39,4% d'amino-4 chloro-6 isopropylamino-2 méthylthio-5 pyrimidine et 5,5% d'isopropylamino-4 amino-6 chloro-2 méthylthio-5 pyrimidine.

Exemple 4.

Mélange des trois isomères amino-2 chloro-6 méthylamino-4 méthylthio-5 pyrimidine, amino-4 chloro-6 méthylamino-2 méthylthio-5 pyrimidine et amino-6 chloro-2 méthylamino-4 méthylthio-5 pyrimidine.

La synthèse est réalisée en deux étapes.

1ère étape:

On opère comme dans la première étape de l'exemple 1 en remplaçant l'éthylamine par 6,2 g de méthylamine en solution à 30, 7% dans l'eau. Après réaction, on obtient par filtration 13 g d'un précipité constitué de dichloro-4,6 méthylamino-2 méthylthio-5 pyrimidine de point de fusion 142°C.

Par concentration sous pression réduite du filtrat et lavage à l'eau du résidu, on obtient 33,4 g d'un produit fondant à 91°C qui, ainsi que le montrent les spectres I.R. et R.M.N., est un mélange de dichloro-4,6 méthylamino-2 méthylthio-5 pyrimidine et de dichloro-2,6 méthylamino-4 méthylthio-5 pyrimidine.

2ème étape:

Dans un autoclave de 500 ml, on introduit 8 g de dichloro-4,6 méthylamino-2 méthylthio-5 pyrimidine, 20,6 g du mélange des deux isomères obtenu à la 1ère étape, 50 g d'ammoniac et 350 g de

méthanol. Après deux heures de chauffage à 130°C, on concentre sous vide la solution obtenue et lave à l'eau le résidu. On obtient ainsi 20,6 g d'un mélange fondant à 118°C Comme le montrent la chromatographie en phase gazeuse et la spectrométrie de masse, ce mélange contient 50,3% d'amino-2 méthylamino-4 chloro-6 méthylthio-5 pyrimidine, 48,3% d'amino-4 méthylamino-2 chloro-6 méthylthio-5 pyrimidine et 1,4% d'amino-6 méthylamino-4 chloro-2 méthylthio-5 pyrimidine.

Exemple 5.

Chloro-6 diamino-2,4 éthylthio-5 pyrimidine. On chauffe à 95°C pendant 5h30 un mélange de 24,6 g d'acide barbiturique, 20 g de diéthylsulfoxyde, 75 ml d'acide acétique glacial et 28 ml d'anhydride acétique. Après refroidissement, on ajoute à froid 175 ml d'eau. Le précipité obtenu est filtré, lavé à l'acétone et séché sous pression réduite. On obtient ainsi 21,7 g de diéthylsulfonium-5 barbiturylide.

Aux 21, 7 g de diéthylsulfonium-5 barbiturylide on ajoute 84,4 g d'oxychlorure de phosphore, 5 ml de diméthylaniline et on chauffe le mélange obtenu à l'ébullition pendant 20 heures. Aprés refroidissement à 60°C, on coule le mélange réactionnel sur de la glace et on agite 1 heure. On fitre le précipité obtenu, on le sèche et on le recristallise dans l'hexane. On obtient ainsi 10 g de trichloro-2,4,6 éthylthio-5 pyrimidine de point de fusion 62—64°C.

Dans un autoclave de 500 ml, on introduit 10 g de trichloro-2,4,6 éthylthio-5 pyrimidine, 17 g d'ammoniac et 100 g de méthanol. Après 2 heures de réaction à 100°C, on filtre le précipité obtenu, on le lave à l'eau et on le sèche sous pression réduite. On obtient ainsi 5,6 g d'un produit fondant à 182°C et consistant essentiellement en chloro-6 diamino-2,4 éthylthio-5 pyrimidine. Ce produit est caractérisé par ses spectres I.R., R.M.N. et de masse.

Exemple 6.

Chloro-6 diamino-2,4 butylthio-5 pyrimidine. Dans un autoclave de 500 ml, on chauffe à 100°C, pendant 2 heures, 120 g de méthanol, 20 g d'ammoniac et 17 g de trichloro-2,4,6 butylthio-5 pyrimidine. Après refroidissement, la solution obtenue est concentrée sous pression réduite. On lave à l'eau le résidu obtenu, et on le recristallise dans le propanol. On obtient ainsi 7 g d'un produit fondant à 129°C et consistant essentiellement en chloro-6 diamino-2,4 butylthio-5 pyrimidine. Ce produit est caractérisé par ses spectres I.R., R.M.N. et de masse.

La trichloro-2,4,6 butylthio-5 pyrimidine utilisée comme produit de départ est préparée selon le procédé décrit à l'exemple 5 pour la préparation de la trichloro-2,4,6 éthylthio-5 pyrimidine, en remplaçant initialement le diéthylsulfoxyde par le dibutylsulfoxyde.

Exemple 7.

Mélange de chloro-6 diamino-2,4 méthylthio-5 pyrimidine (isomère A) et chloro-2 diamino-4,6 méthylthio-5 pyrimidine (isomère B).

Dans un autoclave de 5 litres, on introduit 2,5 litres de méthanol, 250 g d'ammoniac et 250 g de trichloro-2,4,6 méthylthio-5 pyrimidine. On chauffe à 100°C pendant 2 heures et concentre sous pression réduite la solution obtenue. On ajoute de l'éther au résidu obtenu. La partie insoluble dans l'éther est séparée, lavée à l'eau et séchée. On obtient ainsi 153,7 g d'un mélange des isomères A et B, dans lequel l'isomère A est prédominant. Ce mélange fond à 154°C et est caractérisé par ses spectres I.R. et R.M.N.

Exemple 8.

Amino-4 chloro-6 diéthylamino-2 méthylthio-5 pyrimidine.

Dans un ballon de 500 ml d'un réfrigérant, on chauffe à l'ébullition pendant 3 heures 100 g de toluène, 23 g de trichloro-2,4,6 méthylthio-5 pyrimidine et 10,1 g de triéthylamine. Par concentration sous pression réduite de la solution obtenue, on obtient un résidu qui est introduit dans un autoclave de 500 ml avec 350 ml de méthanol et 50 g d'ammoniac. La solution obtenue aprés 2 heures de réaction à 130°C est concentrée à nouveau sous pression réduite. On obtient un résidu qu'on lave à l'eau et que l'on recristallise dans un mélange eau-alcool. On obtient ainsi 17,5 g d'amino-4 chloro-6 diethylamino-2 méthylthio-5 pyrimidine de point de fusion 68°C, qui est caractérisée par ses spectres I.R. et R.M.N.

Exemple 9.

Méthylamino-2 amino-4 chloro-6 méthylthio-5 pyrimidine.

Ce composé est obtenu suivant le mode opératoire de l'exemple 1, en remplaçant dans la première étape l'éthylamine par la méthylamine. Il fond à 205°C et est caractérisé par ses spectres I.R. et R.M.N.

Exemple 10.

Acétylamino-4 diéthylamino-2 chloro-6 méthylthio-5 pyrimidine.

Dans un ballon de 500 ml muni d'un réfrigérant et d'une agitation, on place 250 ml d'acide acétique et 25 g d'amino-4 chloro-6 diéthylamino-2 méthylthio-5 pyrimidine préparée comme indiqué

à l'exemple 8. On chauffe jusqu'à 50°C. Puis on introduit progressivement 50 ml d'anhydride acétique. Après quoi on chauffe 30 minutes à reflux. Puis on évapore sous vide et reprend le résidu à l'eau trois fois pour hydrolyser l'excès d'anhydride acétique.

Le produit brut obtenu est ensuite recristallisé dans l'éthanol. On obtient ainsi un produit qui fond à 72—73°C, dont l'analyse par R.M.N. et I.R. confirme qu'il s'agit de l'acétylamino-4 chloro-6 diéthylamino-2 méthylthio-5 pyrimidine.

Exemple 11.

Préparation d'un mélange de diamino-2,4 chloro-6 méthylthio-5 pyrimidine (isomère A) et de diamino-4,6 chloro-2 méthylthio-5 pyrimidine (isomère B).

Dans un autoclave de 5 litres, on introduit 1300 g de trichloro-2,4,6 méthylthio-5 pyrimidine, 1700 ml d'isopropanol et 495 g d'ammoniac. On chauffe pendant 5 heures à 100°C. Après refroidissement à la température ambiante, on recueille par filtration le précipité formé, on le lave avec 700 ml d'isopropanol puis avec de l'eau, on le sèche. On obtient ainsi 1010 g d'un mélange de diamino-2,4 chloro-6 méthylthio-5 pyrimdine (isomère A) et de diamino-4,6 chloro-2 méthylthio-5 pyrimidine (isomère B), ce qui correspond à un rendement de 93,6% par rapport à la trichloro-2,4,6 méthylthio-5 pyrimidine de départ.

Ce mélange fond à 160°C. Son analyse par chromatographie en couche mince sur silice (élution par un mélange chloroforme/méthanol 90/10), par chromatographie en phase gazeuse couplée avec la spectrométrie de masse, par spectrométrie dans l'infra-rouge et par résonance magnétique nucléaire du carbone 13 montre qu'il contient approximativement 89% d'isomère A et 11% d'isomère B.

Exemple 12.

Préparation de la diamino-2,4 chloro-6 méthylthio-5 pyrimidine (isomère A).

10 g du mélange obtenu à l'exemple 11 sont dissous dans 85 ml d'acide chlorhydrique concentré. A la solution obtenue on ajoute progressivement 55 ml d'eau. Le précipité formé est filtré, lavé à l'eau et séché. On obtient ainsi 2,9 g d'un produit qui est constitué essentiellement par l'isomère A.

Exemple 13.

Préparation d'un mélange diamino-2,4 chloro-6 méthylthio-5 pyrimidine (isomère A) + diamino-4,6 chloro-2 méthylthio-5 pyrimidine (isomère B) enrichi en isomère B.

1ère étape:

Dans 500 ml d'eau contenant 1,2 g de PLURONIC L 92 (agent tensio-actif non ionique constitué par un copolymère d'oxyde d'éthylène et d'oxyde de propylène) on disperse par agitation 46 g de trichloro-2,4,6 méthylthio-5 pyrimidine finement broyée. Puis on introduit en 10 minutes 170 g d'une solution aqueuse d'ammoniac à 20%, en maintenant la température à 5°C. On laisse ensuite une nuit à la température ambiante, puis on filtre le précipité formé et le lave à l'eau. On recueille ainsi 42 g d'un produit qui est un mélange des deux composés isomères dichloro-4,6 amino-2 méthylthio-5 pyrimidine et dichloro-2,6 amino-4 méthylthio-5 pyrimidine, ainsi que le montre en particulier l'analyse par résonance magnétique nucléaire du carbone 13.

2ème étape:

40 g du mélange obtenu dans la 1ère étape sont dissous dans 700 ml d'acide chlorhydrique concentré. A la solution obtenue on ajoute 200 ml d'eau. Il se forme un précipité a que l'on sépare par filtration. On ajoute au filtrat 120 ml d'eau. Il se forme un nouveau précipité b que l'on sépare par filtration. On ajoute enfin au filtrat 260 ml d'eau puis 200 ml d'une solution

$$\frac{N}{10}$$

d'hydroxyde de sodium. Il se forme un précipité c que l'on sépare par filtration.

Le précipité a (poids 6 g) est constitué essentiellement du composé dichloro-4,6 amino-2 méthylthio-5-pyrimidine. Le précipité c (poids 17,2 g) est constitué du composé dichloro-2,6 amino-4 méthylthio-5 pyrimidine.

3ème étape:

Dans un autoclave on introduit 16,5 g du précipité c obtenu à la 2ème étape, 150 ml d'isopropanol et 17 g d'ammoniac. On chauffe à 100°C pendant 3 heures et 15 minutes. Après refroidissement on filtre le précipité formé. On obtient ainsi 11,3 g d'un produit qui est un mélange des deux isomères A et B. La teneur en isomère B du mélange est de 20%.

Exemple 14.

Préparation de la diamino-2,4 chloro-6 méthylthio-5 pyrimidine (isomère A) et de la diamino-4,6 chloro-2 méthylthio-5 pyrimidine (isomère B) pures.

Les isomères A et B sont séparés par chromatographie liquide préparative à partir du mélange obtenu à la 3ème étape de l'exemple 13.

Le mélange est mis en solution dans du chloroforme additionné de 2,5% d'éthanol et la solution est introduite en tête d'une colonne de longueur 25 cm et diamètre intérieur 22 mm, remplie d'un gel de silice de granulométrie 5 $\mu$ connu sous la dénomination commerciale LICHROSORB Si 60 (produit commercialisé par la société Merck). On élue avec du chloroforme additioné de 2,5% d'éthanol. Les fractions recueillies en queue de colonne à l'aide d'un collecteur de fractions sont évaporées. On obtient ainsi 2,1 g de chloro-6 diamino-2,4 méthylthio-5 pyrimidine, dont le point de fusion est 171°C, et 0,9 g de chloro-2 diamino-4,6 méthylthio-5 pyrimidine, dont le point de fusion est 270°C.

Exemple 15.

Dans cet exemple, les produits selon l'invention sont formulés sous forme de suspensions aqueuses contenant 5% d'un tensio-actif dénommé "TWEEN 20".

Les quantités de suspensions appliquées équivalent à 1000 l/ha, et les dilutions réalisées sont calculées de façon à apporter les quantités de matière active suivantes:

$$D_1 = 2,5 \text{ mg/ha}$$

$$D_2 = 10 \text{ kg/ha}$$

Les suspensions sont appliquées par pulvérisation soit sur plantes âgées de 10 jours, ce qui permet d'étudier l'action de post-levée des produits, soit sur semences déposées à la surface du sol, ce qui permet d'étudier l'action de pré-levée. Ces semences sont recouvertes de 2 cm de terre juste après l'application.

Les plantes et graines sont disposées dans des conteneurs en plastique de 18 × 12 × 5 cm remplis d'une terre standard composée de 3 parties de sable, 1 partie de terreau et 1 partie d'argile. Après traitement, les conteneurs sont disposés sur une tablette à irrigation automatique dans une serre maintenue à 22°C et à taux d'hygrométrie de 70%.

Les plantes soumises aux essais sont le blé TRITICUM SP, le haricot PHASEOLUS SP, la betterave BETA SP, la moutarde SINAPIS SP, le pissenlit TARAXACUM SP et le maïs ZEA SP.

On relève les résultats 14 jours après le traitment pour le essais de post-levée et 21 jours après le traitement pour les essais de pré-levée.

Les résultats sont rassemblés dans le tableau I. Dans ce tableau l'efficacité herbicide des composés selon l'invention vis-à-vis des plantes testées est exprimée par un chiffre qui représente le pourcentage de destruction des plantes dans les lots traités. Ce pourcentage est évalué en prenant comme référence les plantes de lots témoins non traités. Le chiffre O indique donc que l'état des plantes est le même dans les lots traités et dans les lots témoins, le chiffre 100 que les plantes sont entièrement détruites dans les lots traités, ce qui correspond à l'efficacité maximum.

Exemple 16.

On conduit les essais comme à l'exemple 15. Seules changent les doses de matière active appliquées. Ces doses sont les suivantes:

$$D_1 = 0,312 \text{ kg/ha}$$

$$D_2 = 0,625 \text{ kg/ha}$$

$$D_3 = 1,25 \text{ kg/ha}$$

$$D_4 = 2,5 \text{ kg/ha}$$

$$D_2 = 5 \text{ kg/ha}$$

Les résultats sont rassemblés dans les tableaux II et III. Les chiffres figurant dans ces tableaux représentent les énergies végétatives des plantes des lots traités, exprimées en pourcentage de l'énergie végétative des plantes des témoins non traités. Le chiffre 100 indique donc que l'énergie végétative des plantes des lots traités est indentique á celle des plantes des témoins, le chiffre 0 que les plantes sont entièrement détruites dans les lots traités.

Dans les tableaux II et III figurent également les résultats relatifs à un herbicide de référence (chlortoluron).

Exemple 17.

On conduit les essais comme à l'exemple 15, avec les doses suivantes de matière active:

$D_1 = 0{,}312$ kg/ha

$D_2 = 0{,}625$ kg/ha

$D_3 = 1{,}25$ kg/ha

$D_4 = 2{,}5$ kg/ha

Les plantes soumises aux essais sont le blé TRITICUM SP, l'orge ORDEUM SP, l'avoine AVENA SP, le riz ORYZA SP, le coton GOSSYPIUM SP, le sétaire SETARIA SP, le panic PANICUM SP, la digitaire PASPALUM SP et le soja. Le composé selon l'invention testé est celui de l'exemple 7.

Les résultats obtenus sont rassemblés dans le tableau IV. La signification des chiffres du tableau IV est la même que celle des chiffres du tableau I. Le chiffre O indique que l'état des plantes est le même dans lots traités dans les lots témoins, le chiffre 100 que les plantes sont entièrement détruites dans les lots traités.

Exemple 18.

Le produit de l'exemple 7 est appliqué en plein champ, par pulvérisation, sur les plantes cultivées (blé, courgette, orge, fève, soja, tournesol, colza, maïs, avoine, pois, tomate) et les plantes advantices indésirables (chenopode, amarante, morelle, mercuriale, laiteron, liseron, seneçon, sétaire), soit en prélevée des plantes immédiatement après les semis, soit en post-levée des plantes 15 jours après les semis. Les doses en produit appliquées sont 2,5 ou 5 kg/ha.

Les résultats sont notés 7, 14 et 100 jours après le traitement (J + 7; 7 + 14; j + 100). Ces résultats sont consignés dans le tableau V. Les chiffres figurant dans ce tableau indiquent, dans le cas plantes cultivées, l'énergie végétative des plantes des parcelles traitées par rapport à celle des plantes des parcelles témoins non traitées et, dans le cas des plantes adventices, le pourcentage de destruction des plantes dans les parcelles traitées, ce pourcentage étant évalué en prenant comme référence les plantes de parcelles témoins non traitées.

Pour une plante cultivée, la note 100 signifie donc que l'énergie végétative de la plante est la même dans les parcelles traitées et dans les parcelles témoins et la note O que plante est entièrement détruite dans les parcelles traitées.

Pour une plante adventice, la note O signifie que l'état de la plante est le même dans les parcelles traitées et les parcelles témoins et la note 100 que la plante est entièrement détruite dans les parcelles traitées.

Exemple 19.

Le produit de l'exemple 7 est appliqué parpulvérisation sur des cultures de blé d'automne de la variété Lutin, à différents stades de la culture (pré-levée, 3 feuilles, tallage, fin de tallage). Les doses en produit appliquées sont 0,625 ou 1,25 kg/ha.

20, 22, 35, 65 et 100 jours après le traitement suivant les cav (voir à ce sujet le tableau VI), on examine l'effet du traitement d'une part sur la plante cultivée (blé), d'autre part sur les plantes adventices indésirables (véronique, paturin, vulpin, mouron, capselle).

Dans aucun cas on n'a enregistré de phytotoxicité vis-à-vis du blé. Dans le tableau VI sont rassemblés les résultats relatifs au taux de destruction des plantes adventices. La note O correspond à une plante indemne, la note 100 à une plante entièrement détruite.

TABLEAU I

EFFICACITE EN POURCENTAGE DE DESTRUCTION

| Produit de l'exemple | Post-levée | | | | | | | | | | | | Pre-levée | | | | | | | | | | | |
|---|---|---|---|---|---|---|---|---|---|---|---|---|---|---|---|---|---|---|---|---|---|---|---|---|
| | $D_1$ = 2,5 kg/ha | | | | | | $D_2$ = 10 kg/ha | | | | | | $D_1$ = 2,5 kg/ha | | | | | | $D_2$ = 10 kg/ha | | | | | |
| | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma |
| 1 | 70 | 100 | – | 100 | 100 | 70 | 75 | 100 | – | 100 | 100 | 100 | 0 | 0 | 0 | 0 | 0 | 0 | 15 | 50 | 100 | 100 | 100 | 0 |
| 2 | 15 | 75 | – | 100 | 100 | 50 | 100 | 100 | – | 100 | 100 | 70 | 15 | 0 | 100 | 100 | 100 | 0 | 50 | 50 | 100 | 100 | 100 | 10 |
| 3 | 20 | 70 | – | 100 | 100 | 70 | 70 | 50 | – | 100 | 100 | 100 | 0 | 0 | 100 | 100 | 100 | 0 | 50 | 50 | 100 | 100 | 100 | 0 |
| 4 | 20 | 50 | – | 100 | 100 | 15 | 50 | 50 | – | 100 | 100 | 15 | 0 | 0 | 100 | 10 | 100 | 0 | 0 | 0 | 100 | 100 | 100 | 0 |
| 5 | 20 | 50 | 100 | 100 | 100 | 15 | 50 | 70 | 100 | 100 | 100 | 70 | – | – | – | – | – | – | – | – | – | – | – | – |
| 6 | 15 | 0 | 100 | 100 | 100 | 0 | 60 | 0 | 100 | 100 | 100 | 0 | – | – | – | – | – | – | – | – | – | – | – | – |
| 7 | 15 | 100 | 100 | 100 | 100 | 0 | 100 | 100 | 100 | 100 | 100 | 15 | 15 | 0 | 100 | 20 | 100 | 0 | 45 | 80 | 100 | 100 | 100 | 0 |
| 8 | 100 | 100 | 100 | 100 | 100 | 50 | 100 | 100 | 100 | 100 | 100 | 100 | 50 | 100 | 100 | 100 | 100 | 0 | 75 | 75 | 100 | 100 | 100 | 0 |
| 9 | 70 | 75 | 100 | 100 | – | 50 | 75 | 100 | 100 | 100 | – | 60 | 50 | 70 | 10 | 75 | – | 0 | 100 | 100 | 100 | 100 | – | 0 |
| 10 | 50 | 80 | 100 | 100 | 100 | 0 | 100 | 90 | 100 | 100 | 100 | 50 | 0 | 0 | 100 | 100 | 100 | 0 | 0 | 0 | 100 | 100 | 100 | 0 |

TABLEAU II

APPLICATION DE POST-LEVEE

ENERGIE VEGETATIVE EN POURCENT DES TEMOINS

| Produit de l'exemple | $D_1$ = 0,312 kg/ha | | | | | | $D_2$ = 0,625 kg/ha | | | | | | $D_3$ = 1,25 kg/ha | | | | | | $D_4$ = 2,5 kg/ha | | | | | | $D_5$ = 5 kg/ha | | | | | |
|---|---|---|---|---|---|---|---|---|---|---|---|---|---|---|---|---|---|---|---|---|---|---|---|---|---|---|---|---|---|---|
| | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma |
| 1 | – | – | – | – | – | – | 70 | 95 | 0 | 0 | 0 | 100 | 70 | 60 | 0 | 0 | 0 | 91 | 60 | 80 | 0 | 0 | 0 | 82 | 25 | 47 | 0 | 0 | 0 | 80 |
| 2 | – | – | – | – | – | – | 100 | 100 | 75 | 82 | 57 | 100 | 100 | 100 | 17 | 27 | 0 | 100 | 100 | 100 | 12 | 4 | 0 | 100 | 70 | 97 | 0 | 0 | 0 | 100 |
| 3 | – | – | – | – | – | – | 52 | 52 | 0 | 0 | 0 | 75 | 33 | 29 | 0 | 0 | 0 | 77 | 8 | 16 | 0 | 0 | 0 | 34 | 27 | 4 | 0 | 0 | 0 | 34 |
| 4 | – | – | – | – | – | – | 92 | 100 | 5 | 5 | 0 | 92 | 95 | 95 | 0 | 0 | 0 | 85 | 85 | 100 | 0 | 0 | 0 | 80 | 78 | 88 | 0 | 0 | 0 | 65 |
| 7 | 84 | 38 | 8 | 46 | 76 | 100 | 24 | 5 | 0 | 0 | 0 | 100 | 5 | 0 | 0 | 0 | 0 | 100 | 0 | 0 | 0 | 0 | 0 | 100 | 0 | 0 | 0 | 0 | 0 | 0 |
| 8 | 90 | 58 | 0 | 5 | 0 | 91 | 73 | 40 | 0 | 0 | 0 | 89 | 58 | 16 | 0 | 0 | 0 | 90 | 33 | 5 | 0 | 0 | 0 | 64 | – | – | – | – | – | – |
| 10 | 50 | 40 | 0 | 40 | 0 | 100 | 40 | 30 | 0 | 20 | 0 | 100 | 30 | 10 | 0 | 10 | 0 | 100 | 10 | 0 | 0 | 0 | 0 | 100 | – | – | – | – | – | – |
| Chlortoluron (herbicide de référence) | | | | | | | | | | | | | 87,5 | 22,5 | 17,5 | 0 | 0 | 17,5 | 0,5 | 17,5 | 0 | 0 | 0 | 52,5 | | | | | | |

TABLEAU III

APPLICATION DE PRE-LEVEE

ENERGIE VEGETATIVE EN POURCENT DES TEMOINS

| Produit de l'exemple | $D_1$ = 0,312 kg/ha | | | | | | $D_2$ = 0,625 kg/ha | | | | | | $D_3$ = 1,25 kg/ha | | | | | | $D_4$ = 2,5 kg/ha | | | | | | $D_5$ = 5 kg/ha | | | | | |
|---|---|---|---|---|---|---|---|---|---|---|---|---|---|---|---|---|---|---|---|---|---|---|---|---|---|---|---|---|---|---|
| | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma | Bl | Ha | Be | Mo | Pi | Ma |
| 1 | – | – | – | – | – | – | 100 | 100 | 7 | 40 | 0 | 100 | 100 | 100 | 5 | 6 | 0 | 100 | 100 | 100 | 0 | 2 | 0 | 100 | 100 | 100 | 0 | 0 | 0 | 100 |
| 2 | – | – | – | – | – | – | 100 | 100 | 75 | 82 | 57 | 100 | 100 | 100 | 17 | 27 | 0 | 100 | 100 | 100 | 12 | 4 | 0 | 100 | 70 | 97 | 0 | 0 | 0 | 100 |
| 3 | – | – | – | – | – | – | 85 | 100 | 75 | 17 | 35 | 100 | 100 | 100 | 80 | 35 | 17 | 100 | 100 | 72 | 25 | 10 | 20 | 85 | 100 | 100 | 7 | 7 | 0 | 93 |
| 4 | – | – | – | – | – | – | 100 | 100 | 100 | 100 | 100 | 100 | 100 | 100 | 75 | 100 | 25 | 100 | 100 | 95 | 100 | 80 | 20 | 100 | 100 | 100 | 75 | 48 | 5 | 100 |
| 7 | 70 | 50 | 6 | 18 | 2 | 100 | 94 | 32 | 1 | 0 | 0 | 100 | 76 | 13 | 0 | 0 | 0 | 100 | 82 | 9 | 0 | 0 | 0 | 100 | 74 | 23 | 0 | 0 | 0 | 86 |
| 8 | 100 | 100 | 0 | 0 | 0 | 100 | 100 | 90 | 0 | 0 | 0 | 80 | 48 | 21 | 0 | 0 | 0 | 78 | 25 | 10 | 0 | 0 | 0 | 73 | – | – | – | – | – | – |
| Chlortoluron | – | – | – | – | – | – | – | – | – | – | – | – | 100 | 85 | 100 | 85 | 12,5 | 100 | 100 | 80 | 78 | 78 | 5 | 95 | – | – | – | – | – | – |

TABLEAU IV

POURCENTAGE DE DESTRUCTION DES PLANTES

APPLICATION DE PRE-LEVEE

| Produit de l'exemple 7 dose en kg/ha | PLANTES TRAITEES | | | | | | | | |
|---|---|---|---|---|---|---|---|---|---|
| | BLE | ORGE | AVOINE | SOJA | RIZ | COTON | PANIC | SETAIRE | DIGITAIRE |
| $D_1$ = 0,312 | 0 | 0 | 0 | 0 | 0 | 0 | 0 | 0 | 0 |
| $D_2$ = 0,625 | 0 | 0 | 8 | 0 | 0 | 0 | 0 | 0 | 0 |
| $D_3$ = 1,25 | 0 | 0 | 31 | 5 | 0 | 0 | 12 | 10 | 9 |
| $D_4$ = 2,5 | 0 | 10 | 52 | 65 | 0 | 0 | 22 | 40 | 25 |

APPLICATION DE POST-LEVEE

| Produit de l'exemple 7 dose en kg/ha | PLANTES TRAITEES | | | | | | | | |
|---|---|---|---|---|---|---|---|---|---|
| | BLE | ORGE | AVOINE | SOJA | RIZ | COTON | PANIC | SETAIRE | DIGITAIRE |
| $D_1$ = 0,312 | 10 | 11 | 32 | 100 | 32 | 33 | 100 | 92 | 47 |
| $D_2$ = 0,625 | 40 | 40 | 50 | 100 | 30 | 55 | 100 | 100 | 67 |
| $D_3$ = 1,25 | 32 | 47 | 65 | 100 | 30 | 87 | 100 | 100 | 75 |
| $D_4$ = 2,5 | 47 | 82 | 95 | 100 | 40 | 90 | 100 | 100 | 100 |

TABLEAU V

| Produit de l'exemple | Doses : | Dates de notation | PLANTE CULTIVEE / ENERGIE VEGETATIVE | | | | | | | | | | | PLANTE ADVENTICE / POURCENTAGE DE DESTRUCTION | | | | | | | |
|---|---|---|---|---|---|---|---|---|---|---|---|---|---|---|---|---|---|---|---|---|---|
| | | | BLE | COURGETTE | ORGE | FEVE | SOJA | TOURNESOL | COLZA | MAIS | AVOINE | POIS | TOMATE | CHENOPODE | AMARANTE | MORELLE | MERCURIALE | LAITERON | LISERON | SENECON | SETAIRE |
| 7 | 2,5 kg/ha | J + 14 | 100 | 90 | 100 | — | 90 | 80 | 100 | 100 | 100 | 100 | 80 | 100 | 100 | 100 | 100 | 100 | 20 | 100 | — |
| | | J + 100 | 0 | 0 | 0 | — | 0 | 20 | 0 | 100 | 0 | 0 | 0 | 80 | 100 | 100 | 100 | 100 | 20 | 100 | 30 |
| | 5 kg/ha | J + 14 | 70 | 50 | 20 | 90 | 0 | 30 | 0 | 90 | — | 70 | 100 | 90 | 100 | 100 | 100 | 100 | 80 | 100 | — |
| | | J + 100 | 0 | 0 | 0 | 0 | 0 | 0 | 0 | 100 | — | 0 | 0 | 100 | 100 | 100 | 100 | 100 | 100 | 100 | 100 |
| | 2,5 kg/ha | J + 7 | 50 | 20 | 70 | 60 | 0 | 10 | 10 | 60 | 50 | 50 | 40 | 100 | 100 | 100 | 100 | 100 | 10 | 100 | — |
| | | J + 14 | 80 | 0 | 60 | 100 | 0 | 30 | 30 | 80 | 70 | 50 | 50 | 100 | 100 | 100 | 100 | 100 | 30 | 100 | — |
| | | J + 100 | 60 | 0 | 0 | 0 | 0 | 0 | 0 | 100 | 0 | 0 | 0 | 100 | 100 | 100 | 100 | 100 | 50 | 100 | 60 |
| | 5 kg/ha | J + 7 | 50 | 10 | 50 | — | 0 | 0 | 0 | 50 | 40 | 0 | 35 | 100 | 100 | 100 | 100 | 100 | — | 100 | — |
| | | J + 14 | 80 | 5 | 60 | 0 | 0 | 0 | 0 | 100 | 60 | 0 | 15 | 100 | 100 | 100 | 100 | 100 | — | 100 | — |
| | | J + 100 | 0 | 0 | 0 | 0 | 0 | 0 | 0 | 100 | 0 | 0 | 0 | 100 | 100 | 100 | 100 | 100 | — | 100 | — |

TABLEAU VI

POURCENTAGE DE DESTRUCTION DES PLANTES

| STADES du BLE | DOSES en kg/ha | DATE de NOTATION | PLANTES ADVENTICES | | | | |
|---|---|---|---|---|---|---|---|
| | | | VERONIQUE (Véronica) | PATURIN (Poa) | VULPIN (Alopecurus) | MOURON (Stellaria) | CAPSELLE (Capsella) |
| PRE-LEVEE | 1,25 | J + 35 | 86 | – | 47 | 100 | – |
| | | J + 65 | 100 | 80 | 52 | 100 | – |
| | | J + 100 | 98 | 88 | 62 | 100 | 100 |
| 3-FEUILLES | 0,625 | J + 22 | 86 | 41 | 63 | 78 | 80 |
| | | J + 35 | 91 | – | 70 | 70 | 100 |
| | 1,25 | J + 22 | 91 | 100 | 70 | 100 | 100 |
| | | J + 35 | 98 | – | 82 | 100 | 100 |
| TALLAGE | 1,25 | J + 20 | 85 | 70 | – | 80 | 90 |
| FIN TALLAGE | 1,25 | J + 22 | 80 | – | – | 80 | 90 |

**Revendications**

1. Composés de formule:

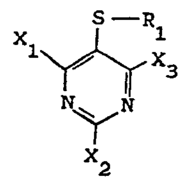

(I)

dans laquelle $R_1$ est un groupe alkyle ayant 1 à 5 atomes de carbone, $X_1$ est un atome de chlore, $X_2$ est un groupe

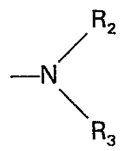

dans lequel $R_2$ est un atome d'hydrogène ou un groupe alkyle ayant 1 à 5 atomes de carbone, $R_3$ est un atome d'hydrogène ou un groupe alkyle ayant 1 à 5 atomes de carbone et peut être en outre, lorsque $R_2$ est un atome d'hydrogène, un groupe

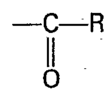

dans lequel R est un atome d'hydrogène ou un groupe alkyle de 1 à 5 atomes de carbone, $X_3$ est un groupe

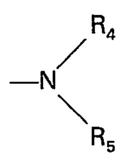

dans lequel $R_4$ est un atome d'hydrogène et $R_5$ est un atome d'hydrogène ou un groupe

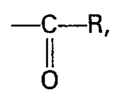

R étant tel que défini ci-dessus, et leurs sels avec les acides minéraux ou organiques.

2. Composés selon la revendication 1, caractérisé en ce que $X_2$ est un groupe méthylamino, éthylamino, isopropylamino, diéthylamino ou amino, et $X_3$ est un groupe $NH_2$ ou

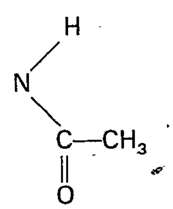

3. Le composé diamino-2,4 chloro-6 méthylthio-5 pyrimidine.

4. Le composé amino-4 diéthylamino-2 chloro-6 méthylthio-5 pyrimidine.

5. Le composé acétylamino-4 diéthylamino-2 chloro-6 méthylthio-5 pyrimidine.

6. Procédé de préparation des composés de formule (I) dans lesquels $R_3$ et $R_5$ ne sont pas

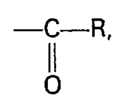

caractérisé en ce qu'on condense une trichloro-2,4,6 alkylthio-5 pyrimidine de formule:

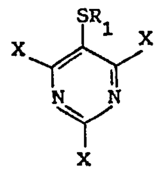

(II)

dans laquelle X est un atome de chlore et $R_1$ est un groupe alkyle ayant 1 à 5 atomes de carbone, avec un composé de formule

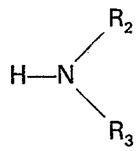

,

dans laquelle $R_2$ et $R_3$ ont les mêmes significations que dans la revendication 1 excepté la signification

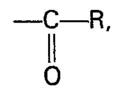

et condense la dichloro-4,6 alkylthio-5 pyrimidine ainsi obtenue avec un composé de formule

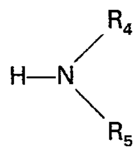

,

dans laquelle $R_4$ et $R_5$ ont les mêmes significations que dans la revendication 1 excepté la signification

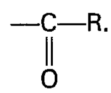

7. Procédé de préparation des composés de formule (I) dans lesquels $X_3$ est un groupe $NH_2$ et $X_2$ est un groupe

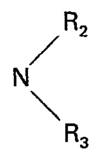

dans lequel $R_2$ et $R_3$ sont des groupes alkyle identiques R', caractérisé en ce qu'on fait réagir une trichloro-2,4,6 alkylthio-5 pyrimidine de formule:

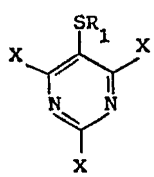

(II)

dans laquelle X est un atome de chlore et $R_1$ est un groupe alkyle ayant 1 à 5 atomes de carbone, avec une amine tertiaire de formule $N(R')_3$ et condense la dichloro-4,6 alkylthio-5 pyrimidine ainsi obtenue avec l'ammoniac.

8. Procédé de préparation des composés de formule (I) dans lesquels $X_2$ et $X_3$ sont des groupes $NH_2$, caractérisé en ce qu'on condense l'ammoniac avec une trichloro-2,4,6 alkylthio-5 pyrimidine de formule:

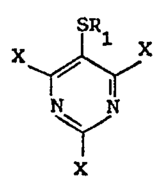

(II)

dans laquelle X est un atome de chlore et $R_1$ est un groupe alkyle ayant 1 à 5 atomes de carbone.

9. Application en tant qu'herbicides des composés définis dans chacune des revendications 1 à 5.

10. Compositions herbicides caractérisées en ce qu'elles contiennent, à titre de matière active, un ou plusieurs composés tels que définis dans chacune des revendications 1 à 5.

11. Compositions herbicides caractérisées en ce qu'elles contiennent, à titre de matière active, un ou plusieurs composés tels que définis dans chacune des revendications 1 à 5 et au moins un autre herbicide.

12. Compositions herbicides selon chacune des revendications 10 et 11, caractérisées en ce que le composé inclus comme matière active est la diéthylamino-2 acétylamino-4 chloro-6 méthylthio-5 pyrimidine.

13. Compositions herbicides selon chacune des revendications 10 et 11, caractérisées en ce que le composé inclus comme matière active est la diamino-2,4 chloro-6 méthylthio-5 pyrimidine.

**Claims**

1. Compounds of the formula:

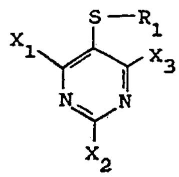

(I)

in which $R_1$ is an alkyl group having 1 to 5 carbon atoms, $X_1$ is a chlorine atom, $X_2$ is a

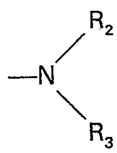

group in which $R_2$ is a hydrogen atom or an alkyl group having 1 to 5 carbon atoms, $R_3$ is a hydrogen atom or an alkyl group having 1 to 5 carbon atoms and in addition, when $R_2$ is a hydrogen atom, may be a

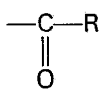

group in which R is a hydrogen atom or an alkyl group having 1 to 5 carbon atoms, $X_3$ is a

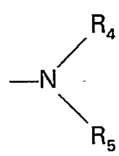

group in which $R_4$ is a hydrogen atom and $R_5$ is a hydrogen atom or a

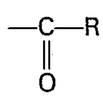

group, R being as defined above, and their salts with mineral or organic acids.

2. Compounds according to claim 1, characterised in that $X_2$ is a methylamino, ethylamino, isopropylamino, diethylamino or amino group, and $X_3$ is a $NH_2$ or

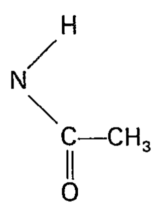

group.

3. The compound 2,4-diamino-6-chloro-5-methylthio pyrimidine.

4. The compound 4-amino-2-diethylamino-6-chloro-5-methylthio pyrimidine.

5. The compound 4-acetylamino-2-diethylamino-6-chloro-5-methylthio pyrimidine.

6. Process for the preparation of the compounds of formula (I) in which $R_3$ and $R_5$ are not

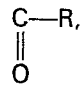

characterised in that a 2,4,6-trichloro-5-alkylthio pyrimidine of the formula:

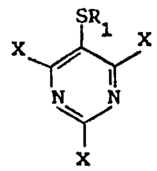

(II)

in which X is a chlorine atom and $R_1$ is an alkyl group having 1 to 5 carbon atoms, is condensed with a compound of the formula

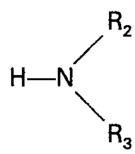 ,

in which $R_2$ and $R_3$ have the same meanings as in claim 1 with the exception of the

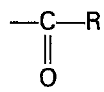

meaning, and the 4,6-dichloro-5-alkylthio pyrimidine thus obtained is condensed with a compound of the formula

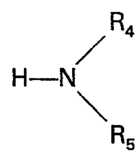 ,

in which $R_4$ and $R_5$ have the same meanings as in claim 1, with the exception of the

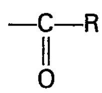

meaning.

7. Process for the preparation of the compounds of formula (I) in which $X_3$ is a $NH_2$ group and $X_2$ is a

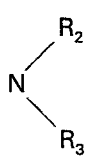

group in which $R_2$ and $R_3$ are identical R′ alkyl groups, characterised in that a 2,4,6-trichloro-5-alkylthio pyrimidine of the formula:

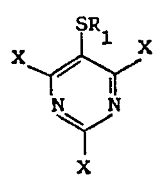

(II)

in which X is a chlorine atom and $R_1$ is an alkyl group having 1 to 5 carbon atoms, is reacted with a tertiary amine of the formula $N(R')_3$, and the 4,6-dichloro-5-alkylthio pyrimidine thus obtained is condensed with ammonia.

8. Process for the preparation of the compounds of formula (I), in which $X_2$ and $X_3$ are $NH_2$ groups, characterised in that ammonia is condensed with a 2,4,6-trichloro-5-alkylthio pyrimidine of the formula:

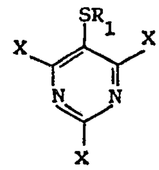

(II)

in which X is a chlorine atom and $R_1$ is an alkyl group having 1 to 5 carbon atoms.

9. Use as herbicides of the compounds defined in each of claims 1 to 5.

10. Herbicidal compositions, characterised in that they contain, as active material, one or more compounds as defined in each of claims 1 to 5.

11. Herbicidal compositions, characterised in that they contain as active material, one or more compounds as defined in each of claims 1 to 5 and at least one other herbicide.

12. Herbicidal compositions according to each of claims 10 to 11, characterised in that the compound included as active material is 2-diethylamino-4-acetylamino-6-chloro-5-methylthio pyrimidine.

13. Herbicidal compositions according to each of claims 10 to 11, characterised in that the compound included as active material is 2,4-diamino-6-chloro-5-methylthio pyrimidine.

**Patentansprüche**

1. Verbindungen der Formel:

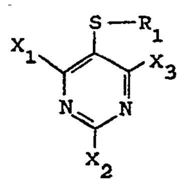

(I)

in der $R_1$ eine Alkylgruppe mit 1 bis 5 Kohlenstoffatomen, $X_1$ ein Chloratom, $X_2$ eine Gruppe

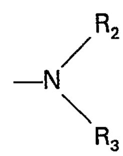

bedeuten, worin $R_2$ ein Wasserstoffatom oder eine Alkylgruppe mit 1 bis 5 Kohlenstoffatomen, und $R_3$ ein Wasserstoffatom oder eine Alkylgruppe mit 1 bis 5 Kohlenstoffatomen bedeuten und $R_3$ außerdem, wenn $R_2$ ein Wasserstoffatom ist, eine Gruppe

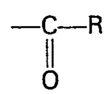

bedeuten kann, in der R ein Wasserstoffatom oder eine Alkylgruppe mit 1 bis 5 Kohlenstoffatomen bedeutet, und $X_3$ eine Gruppe

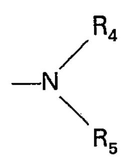

ist, in der $R_4$ ein Wasserstoffatom und $R_5$ ein Wasserstoffatom oder eine Gruppe

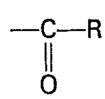

bedeuten, in der R die oben angegebene Bedeutung hat, sowie ihre Salz mit Mineralsäuren oder organischen Säuren.

2. Verbindungen nach Anspruch 1, dadurch gekennzeichnet, daß $X_2$ eine Methylamino-, Äthylamino-, Isopropylamino-, Diäthylamino- oder Aminogruppe und $X_3$ eine Gruppe $—NH_2$ oder

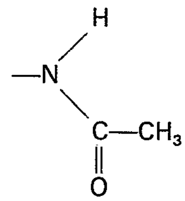

3. 2.4-Diamino-6-chlor-5-methylthiopyrimidin.
4. 4-Amino-2-diäthylamino-6-chlor-5-methylthiopyrimidin.
5. 4-Acetylamino-2-diäthylamino-6-chlor-5-methylthiopyrimidin.
6. Verfahren zur Herstellung der Verbindungen der Formel (I), in denen $R_3$ und $R_5$ nicht

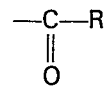

bedeuten, dadurch gekennzeichnet, daß man ein 2.4.6-Trichlor-5-alkylthiopyrimidin der Formel:

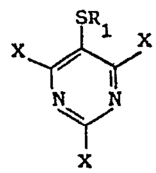

(II)

in der X ein Chloratom und $R_1$ eine Alkylgruppe mit 1 bis 5 Kohlenstoffatomen bedeuten, mit einer Verbindung der Formel

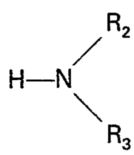 ,

in der $R_2$ und $R_3$ die gleichen Bedeutungen wie in Anspruch 1 mit Ausnahme der Bedeutung

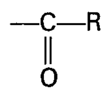

haben, kondensiert und das auf diese Weise erhaltene 4.6-Dichlor-5-alkylthiopyrimidin mit einer Verbindung der Formel

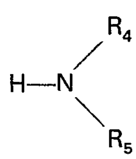

kondensiert, in der $R_4$ und $R_5$ die gleichen Bedeutungen wie in Anspruch 1 mit Ausnahme der Bedeutung

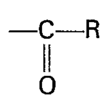

aufweisen.

7. Verfahren zur Herstellung der Verbindungen der Formel (I), in denen $X_3$ eine Gruppe $—NH_2$ und $X_2$ eine Gruppe

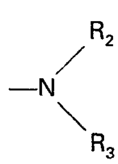

bedeuten, in der $R_2$ und $R_3$ identische Alkylgruppe R' sind, dadurch gekennzeichnet, daß man ein 2.4.6-Trichlor-5-alkylthiopyrimidin der Formel:

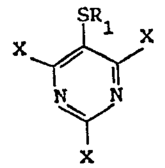

(II)

in der X ein Chloratom und $R_1$ eine Alkylgruppe mit 1 bis 5 Kohlenstoffatomen sind, mit einem tertiären Amin der Formel $N(R')_3$ zur Umsetzung bringt und das auf diese Weise erhaltene 4.6-Dichlor-5-alkylthiopyrimidin mit Ammoniak kondensiert.

8. Verfahren zur Herstellung von Verbindungen der Formel (I), in denen $X_2$ und $X_3$ $NH_2$-Gruppen bedeuten, dadurch gekennzeichnet, daß man Ammoniak mit einem 2.4.6-Trichlor-5-alkylthiopyrimidin der Formel:

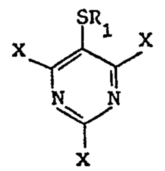

(II)

kondensiert, in der X ein Chloratom und $R_1$ eine Alkylgruppe mit 1 bis 5 Kohlenstoffatomen bedeuten.

9. Verwendung der Verbindungen gemäß den Ansprüchen 1 bis 5 als Herbizide.

10. Herbizide Zubereitungen, dadurch gekennzeichnet, daß sie als Wirkstoff eine oder mehrere Verbindungen gemäß den Ansprüchen 1 bis 5 enthalten.

11. Herbizide Zubereitungen, dadurch gekennzeichnet, daß sie als Wirkstoff eine oder mehrere Verbindungen gemäß den Ansprüchen 1 bis 5 und mindestens ein anderes Herbizid enthalten.

12. Herbizide Zubereitungen nach einem der Ansprüche 10 und 11, dadurch gekennzeichnet, daß sie als Wirkstoff 2-Diäthylamino-4-acetylamino-6-chlor-5-methylthiopyrimidin enthalten.

13. Herbizide Zubereitungen nach einem der Ansprüche 10 und 11, dadurch gekennzeichnet, daß sie als Wirkstoff 2.4-Diamino-6-chlor-5-methylthiopyrimidin enthalten.